# EUROPEAN PATENT APPLICATION

(11) **EP 2 050 475 A2**
(43) Date of publication of application: **22.04.2009**
(21) Application number: 09150677.4
(22) Date of filing: 06.09.2006
(51) Int. Cl.: A61L 31/18

(54) **Medical devices incorporating radio-opaque and biocompatible coatings**

(30) Priority: 06.09.2005 US 714818 P; 06.12.2005 US 295780
(62) Divisional of application: 06814188.6
(71) Applicant: Honeywell International Inc., Morristown, NJ 07962 (US)
(72) Inventor: Dalzell, William J, Parish, FL 34219 (US); Heffner, Kenneth H, Largo, FL 33778 (US)
(74) Representative: Buckley, Guy Julian

(57) **Abstract**

Medical devices having radio-opaque and/or biocompatible coatings incorporated therewith

## Description

### Cross-Reference To Related Application

The present non-provisional Application claims the benefit of commonly owned provisional Application having serial number 60/714,818, filed on September 6, 2005, and entitled MEDICAL DEVICES INCORPORATING RADIO-OPAQUE AND BIOCOMPATIBLE COATINGS, which Application is incorporated herein by reference in its entirety.

### Technical Field

The present invention relates generally to radio-opaque and biocompatible coatings for use with medical devices. More particularly, the present invention relates to methods of incorporating such radio-opaque and/or biocompatible coatings with medical devices such as implantable medical devices.

### Background

Specialized coatings are often used with electronic devices, especially microelectronic devices. One type of specialized coating is used to provide shielding or blocking functions with respect to potentially harmful electromagnetic radiation. For example, shielding can be used to protect an electronic device from external radiation sources and/or help to contain or direct radiation emitted by a source internal to the device itself. Thus, these coatings are often referred to as radio-opaque coatings. Radio-opaque materials in the form of shielding, partitions, or other configurations, as used to contain and control the emissions of radiation sources, for example, are discussed in U.S. Pat. Nos. 6,320,936 and 6,185,279, each of which is incorporated herein by reference in its respective entirety for all purposes.

The use of radiation shielding coatings is particularly desirable with electronic devices such as those to be implanted in human or animal (e.g. mammal) patients. Often these devices include electronic circuitry or the like wherein radiation may induce a degradation pathway for the performance of the device. Typical, implantable medical devices that include such electronic circuitry include pacemakers, defibrillators, nerve stimulators, hearing aids, and drug pumps, for example. It is often desirable to shield this type of implantable medical device from external electromagnetic flux that could cause interference or disrupt the operation of the device. For example, patients having implanted medical devices may be exposed to microwave ovens, cellular phones, or security equipment that uses electromagnetic radiation or energy based signals to scan individuals such as at airports or the like. It may also be desirable to partially or completely prevent implanted devices from emitting radiation or energy. An example would relate to a medical device that is implanted so that it can emit directed radiation in order to destroy or otherwise damage a tumor or other undesired growth. In this regard, the radio-opaque coating is designed to minimize the exposure of healthy tissue to emitted radiation.

In any event, implantable medical devices further need to be biocompatible. Biocompatible generally relates to the ability of such a device to be accepted by human or animal body in which it is implanted. That is, such devices can be implanted so that they do not cause unacceptable inflammation, infection, scarring or act as a base for encapsulation, cyst or cell mass.

### Summary

The present invention provides radio-opaque coatings and/or biocompatible coatings and methods of integrating such coatings with electronic devices such as implantable medical devices, for example. These coatings help to provide radiation shielding and biocompatibility for implantable medical devices. A radio-opaque coating with sufficient shielding properties can be used with medical devices that may include electronic circuitry, actuators, sensors, and/or micro-electromechanical systems that may possess sensitivity to electro-magnetic radiation. Alternatively, miniature medical devices with an imbedded nuclide source capable of delivering a dose lethal to surrounding tissue (e.g., malignant cells) could be strategically coated to minimize the dose level absorbed by healthy, co-resident tissue and reduce co-morbidity of healthy cells in the site-specific treatment of malignant cells. A biocompatible coating can be used to provide biocompatibility together with a radiation blocking coating for such implantable medical devices. In accordance with the present invention, thermal spray techniques can be used to incorporate radio-opaque or biocompatible coatings with implantable medical devices. Using thermal spray methodologies, radio-opaque and biocompatible coatings can be provided even on thermally sensitive components, such as certain implantable medical devices, without undue risk of thermal damage to these devices.

Principles of the present invention may be useful in a wide range of applications such as where radiation or energy may be directed toward or used to otherwise acquire information about a subject. In this regard, radio-opaque coatings of the present invention can help to prevent undesirable electromagnetic radiation, energy, or signals from interfering with the operation of a medical device. Such applications may involve medical imaging systems, implantable medical devices including pacemakers, defibrillators, nerve stimulators, hearing aids, and drug pumps, implantable medical devices that irradiate a desired target with radiation, and security assessment such as by x-ray, magnetometer, or radio-frequency type devices.

Imbedded medical devices with a self contained circuit are sensitive to the processes used to apply coating materials with a high prospect for shielding and biocompatibility. The thermal spray coating methods which are the subject of this invention ensure the stability of the imbedded circuitry while permitting the strategic shielding required to achieve the benefits discussed to this point.

In one aspect of the present invention, a method of integrating a radiation shield with an implantable medical device is provided. The method comprises the steps of providing an implantable medical device and thermally spraying a radio-opaque composition onto a portion of the medical device.

In another aspect of the present invention, a method of integrating a radiation shield with a medical device is provided. The method comprises the steps of providing a medical device, thermally spraying a radio-opaque composition onto a first portion of the medical device, and thermally spraying a biocompatible composition onto a second portion of the medical device.

In another aspect of the present invention, an implantable medical device is provided. The medical device comprises a housing, electronic circuitry substantially enclosed by the housing, a radio-opaque coating substantially surrounding at least a portion of the electronic circuitry, and a biocompatible coating defining at least a portion of an outside surface of the medical device.

In yet another aspect of the present invention, an implantable medical device is provided. The medical device comprises a housing, electronic circuitry substantially enclosed by the housing, a radio-opaque coating deposited on at least a portion of the housing by passing the at least a portion of the housing through a plume of a spray comprising molten particles of a radio-opaque composition, and a biocompatible coating deposited on at least a portion of one or both of the housing and the radio-opaque coating by passing the at least a portion of one or both of the housing and the radio-opaque coating through a plume of a spray comprising molten particles of a biocompatible composition.

### Brief Description of the Drawings

These and other features, aspects, and advantages of the present invention will become better understood with regard to the following description, appended claims, and accompanying drawings where:
Figure 1 is a schematic perspective view of a pacemaker in accordance with the present invention showing in particular a radio-opaque coating provided on a housing of the pacemaker and a biocompatible coating provided on the radio-opaque coating;
Figure 2 is a schematic cross-sectional view of the pacemaker of Figure 1;
Figure 3 shows an illustrative apparatus for creating radio-opaque and biocompatible coatings of the present invention using thermal spray techniques;
Figure 4 is a front view of the thermal spray gun used in Figure 3 showing the nozzle configuration;
Figure 5 is a schematic illustration of an alternative apparatus for creating radio-opaque and biocompatible coatings of the present invention using thermal spray techniques;
Figure 6 is a schematic cross-sectional view of a medical device of the present invention showing in particular a radiation shield that allows radiation to be directed to tissue to be treated with the radiation; and
Figure 7 is a schematic view of another pacemaker in accordance with the present invention showing in particular a radio-opaque coating provided internal to a housing of the pacemaker and a biocompatible coating provided external to the housing.

### Detailed Description

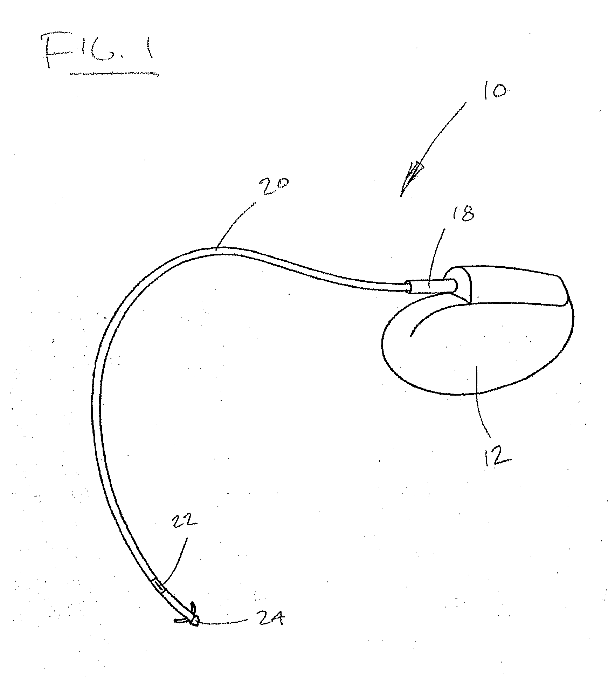

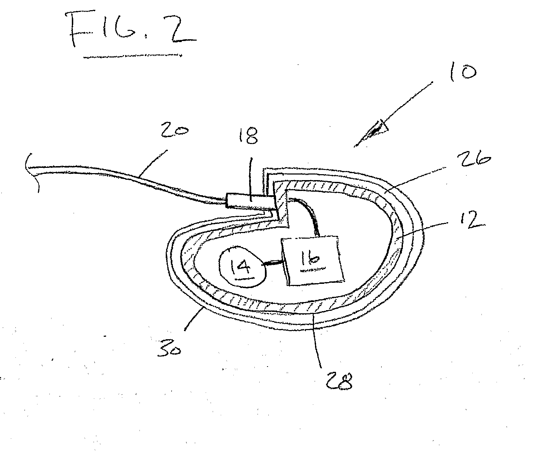

Figures 1 and 2 schematically show an implantable medical device representative of the type in which the present invention may be practiced. In this case, the device illustrated is a cardiac pacemaker 10 having a housing 12, which contains a battery 14 and associated electronic circuitry 16. A connector 18 is coupled to housing 12 of pacemaker 10, and is typically fabricated of epoxy or other plastic. Inserted into the connector 18 is a pacing lead 20, which is in electrical communication with a pulse generator of electronic circuitry 16 within the pacemaker 10, and which serves to deliver pacing pulses to pacing electrodes 22 and 24. To help shield electronic circuitry 16 from incoming radiation, radio-opaque coating 26 providing radiation shielding capabilities is preferably provided on outside surface 28 of housing 12 of pacemaker 10. Radio-opaque coating 26 preferably encloses circuitry 16 by coating substantially the entire outside surface 28 of housing 12 of pacemaker 10. As shown, radio-opaque coating 26 is not provided on connector 18 and pacing lead 20. However, these components may be coated with a radio-opaque coating if desired. In any event, any portion of outside surface 28 and/or an internal surface or portion can be coated to achieve a desired radiation blocking functionality.

Radio-opaque coating 26 may be formed from any material or combination of materials that help provide such a coating with radiation shielding characteristics. Radiation shielding relates to the use of a material(s) that can alter some characteristic of a source of particles and/or photons (such as spectrum, fluence, intensity, or the like) through physical interaction between the atomic structure of the atoms of the material and the incident photons or particles striking the material. The net reduction in such a characteristic of the incident particles or photons and any contribution by secondary radiation can be used to assess the shielding effectiveness of the material. Representative examples of materials with radiation shielding characteristics include elements having an atomic number of 39 or greater, preferably 56 or greater, more preferably 72 or greater, compounds of such elements, alloys incorporating such elements, admixtures incorporating such elements, combinations of these and the like. Elements with low atomic numbers (hydrogen and carbon, for example) also have the capacity to shield radiation effectively. However, it typically takes more material to provide effective shielding. Representative examples of preferred elements include Hf, Ta, W, Re, Os, Ir, Pt, Au, Tl, Pb, Bi, and Ba. Heavier elements and materials incorporating such heavier elements, such as W, are more preferred singly or in combination, as these tend to provide more shielding capability at a given coating thickness than lighter elements. Carbon-based materials such as polyethylene may also be used. Polyethylene, for instance, is a suitable shielding material inasmuch as polyethylene coatings are highly dense due to favorable packing density characteristics. A specific embodiment of radio-opaque coating 26 might include, for example, layers of tungsten, or a combination of tungsten and polyethylene as individual or multiple layers.

The thickness of radio-opaque coating 26 may vary over a wide range. As general guidelines, if the coating is too thin, then the ability of the coating to help shield and/or contain energy may be less than is desired. Accordingly, thickness of coating 26 is preferably determined based on factors such as the composition of coating 26, the type of energy that coating 26 is desired to shield, and the nature of the shielding functionality that is desired, for example. For one exemplary application for isolating an imbedded internal nuclide (β-emitter), radio-opaque coating 26 desirably may have a thickness of at least 10 micrometers, preferably at least 90 micrometers, more preferably at least 1000 micrometers. On the other hand, if the coating is too thick, then the practicality of coating devices may be limited. Accordingly, it is preferred that radio-opaque coating 26 has a thickness of up to 90 micrometers, desirably up to 150 micrometers, more desirably up to 250 micrometers. In this exemplary application, a secondary benefit of the coating is the absorption and attenuation of secondary radiation produced by beta particle decay.

As an option, surface 28 of housing 12 of pacemaker 10 (or any other surface on which radio-opaque coating 26 is desired to be formed) may be primed to improve adhesion. This may be accomplished by physically or chemically altering such a surface via a technique such as corona, etching, ultraviolet, e-beam, x-ray, oxidation, reduction, heating, roughening, or other suitable treatment. Alternatively, a primer coating (not shown) constituting one or more priming materials in one or more priming layers may be used. As an example, a pre-coat of an organic, metallic, or ceramic material could be used to promote adhesion of a tungsten-containing material to a surface of an implantable medical device. Representative examples of primer materials include a highly-filled composite or metal/semi-metal silicates with an intermediate coefficient of thermal expansion (CTE) to ensure that there is relief in the CTE mis-match between the vacuum tube glass and the tungsten metal. These materials can be applied by chemical vapor deposition (CVD) or Radio frequency (RF) sputtering for the silicates or by paint or immersion for the composite pre-coat (e.g. acrylic w/titanium oxide filler).

As another option, radio-opaque coating 26 may receive a post-treatment if desired. For example, radio-opaque coating 26 may be polished following deposition to enhance gloss and surface finish as desired. Radio-opaque coating 26 may be treated as noted above to improve anti-clotting and anti-thromboembolic properties of any subsequent coating such as a biocompatible coating as described below. In other modes of practice, radio-opaque coating 26 may receive a protective overcoat (not shown) to protect radio-opaque coating 26 from damage, oxidation, or the like. Examples of materials that would be suitable to form an overcoat include reactive or ion-planted metals (applied by chemical vapor deposition, combinations of these, and the like).

In accordance with the present invention, pacemaker 10 may also include optional biocompatible coating 30. As used herein, biocompatible or biocompatibility with respect to a coating means that the coating enhances the degree to which a device is biologically non-interfering, inert, and/or passive when implanted as compared to an otherwise identical device lacking the coating. Thus, a biocompatible or biocompatibility coating in accordance with this invention is one that is biologically inert, non-toxic, and of a non-interfering character. As shown, biocompatible coating 30 is provided on radio-opaque coating 26 but can be formed on any desired portion of pacemaker 10. Biocompatible coating 30 may be formed from a wide range of materials. Exemplary materials include bioglass, hydroxyapatite, titanium and titanium alloys, alpha-alumina, stabilized zirconia, apatite ceramics, combinations of these, and the like. Biocompatible coating 30 may comprise any number of layers of any desired materials.

The thickness of biocompatible coating 30 may vary over a wide range. As general guidelines, if the coating is too thin, then the ability of the coating to help provide tissue adhesion and/or minimize inflammatory response may be less than is desired. Accordingly, biocompatible coating 30 desirably may have a thickness of at least 0.01 micrometers, preferably at least 1 micrometer, more preferably at least 2 micrometers. On the other hand, if the coating is too thick, then the structural integrity of the base coat may be affected. Accordingly, it is preferred that biocompatible coating 30 has a thickness of up to 0.1 micrometers, more desirably up to 5 micrometers. Coating 30 may of course have thickness less than 0.01 micrometers and greater than 5 micrometers depending on the material composition, coating technique, desired biocompatible functionality, and/or the particular medical device used.

A surface onto which a biocompatible coating is applied may be treated to improve adhesion in the same manner as described above with respect to radio-opaque coating 26. Also, any desired post surface treatments may be used.

In preferred modes of practice, radio-opaque and biocompatible coatings of the present invention advantageously are formed on surfaces using thermal spray techniques. The use of thermal spray techniques allows such a coating to be formed on a wide range of surfaces, including in particular temperature sensitive or delicate surfaces, such as implantable medical devices. Thermal spraying allows coatings to be formed without unduly thermally damaging such surfaces.

Generally, thermal spraying involves causing a surface or substrate to be coated to pass through a plume of a spray comprising molten particles of the coating composition. In preferred modes of practice, a line of sight coating process uses heat energy to heat the coating material to a molten state. The molten material typically is caused to be atomized or otherwise converted into molten droplets. The molten material is carried to the substrate by a carrier gas or jet. The molten droplets are preferably finely sized. During coating, the substrate is moved in and out of the hot spray to minimize thermal risk to the substrate. The desired coating thickness desirably is built up using multiple passes. The substrate optionally may be thermally coupled to a heat sink and/or chilling media during thermal spraying in order to help carry away thermal energy imparted to the substrate.

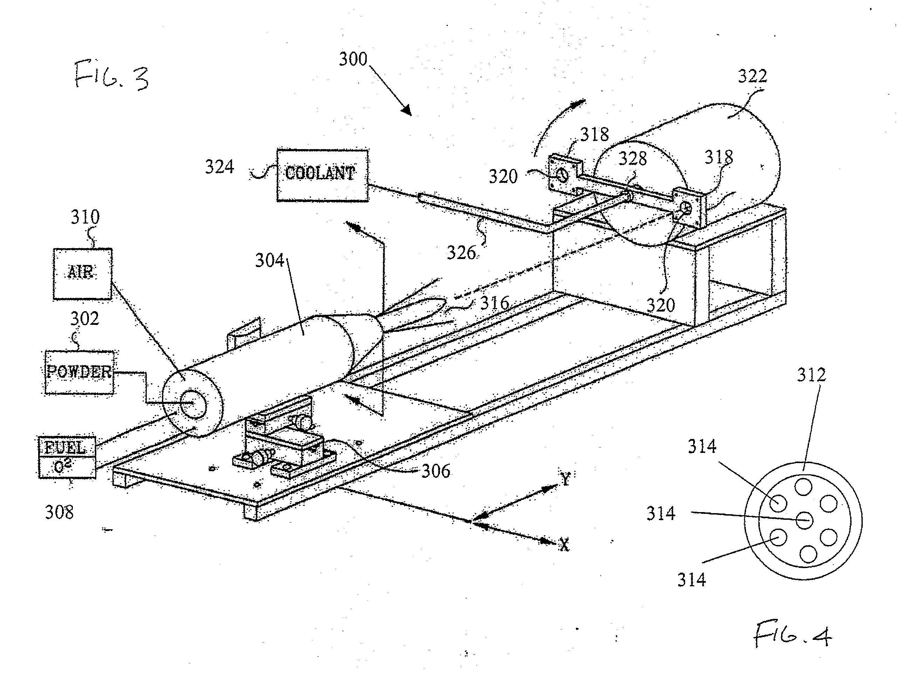

One embodiment of a thermal spray system 300 useful to carry out thermal spraying is illustrated in Figures 3 and 4. Particles, e.g., a fine powder, of a coating composition are supplied from a composition feedstock supply 302 to the thermal spray gun 304. The gun 304 is mounted on an X-Y positioning rack 306. Thermal spray gun 304 may be of a variety of types, including a flame gun, plasma gun, electric arc, gun or the like. For purposes of illustration, spray gun 304 is a flame-type gun. In such an embodiment, fuel and oxygen are supplied to the gun 304 from a fuel/oxidant supply 308 and air is supplied from an air supply 310. The air is ejected through annular nozzle 312, and the flame is emitted from nozzles 314 located centrally inside annular nozzle 312. The air carries entrained particles (not shown), which are melted by the flame 316 as the particles exit the gun 304. The air acts not only as a carrier gas to help transport the molten particles to the substrate (not shown) to be coated, but the air also acts as a nozzle coolant.

The molten particles are aimed at a pair of rotatable arms 318. The arm ends 320 each receive one or more corresponding substrates to be coated. By rotating arms 318, the substrates repeatedly move in an out of the spray plume. In this way, the thermal spray coating can be applied without excessively heating the substrates. Each substrate generally may be planar and may be fixedly mounted to an arm end 320. However, three-dimensional substrates such as pacemakers and other implantable medical devices may also be coated in this way. These would be mounted onto arms 318 so that the three dimensional substrate could be spun in several axis modes while the gun 304 sprays molten material onto the surfaces of the substrate in line of sight fashion.

The arms 318 are rotated by an electric motor 322. A coolant, such as compressed air, is pumped into the arms 318 from a coolant supply 324 through a pipe or hose 326 that connects to a coolant slip ring 328 located generally at the central axis of rotation of arms 318. The coolant flows from the slip ring to coolant passages (not shown) inside arms 318. Those passages desirably extend radially along the interior of arms 318 and each arm end 320.

The arms 318 rotate at a suitable rate, sweeping the mounted substrates through the spray of molten particles. As general guidelines, rotational rates within the range of 1 to 500 rpm, more desirably 300 to 350 rpm would be suitable. With each pass, the coating builds up on the surface(s) of the substrate in line of sight coating fashion. As a practical matter, the deposition of coating material tends to be a small swath along the substrate surfaces in the direction of rotation R, and as the arms 318 rotate. Accordingly, the gun 304 is indexed in the radial direction (X direction) with respect to the arms 318 so that the coating covers the entire surfaces to be coated. The speed of movement in the X direction optionally may be adjusted so that the deposition rate of material onto the substrate is constant. Otherwise, faster moving portions of the surfaces radially farther from the center of rotation may receive less material per unit time than those closer to the center of rotation.

The distance from the gun 304 to the arms 318 also is adjustable in the Y direction. In many embodiments, a desired distance is one at which substrate heating is below a desired threshold, yet the composition is still molten when it impacts the substrate. Thus, if gun 304 were to be too close to a substrate, the substrate might get too hot. If too far, the molten droplets might solidify too much before reaching the substrate surfaces, impairing the quality of the resultant coating.

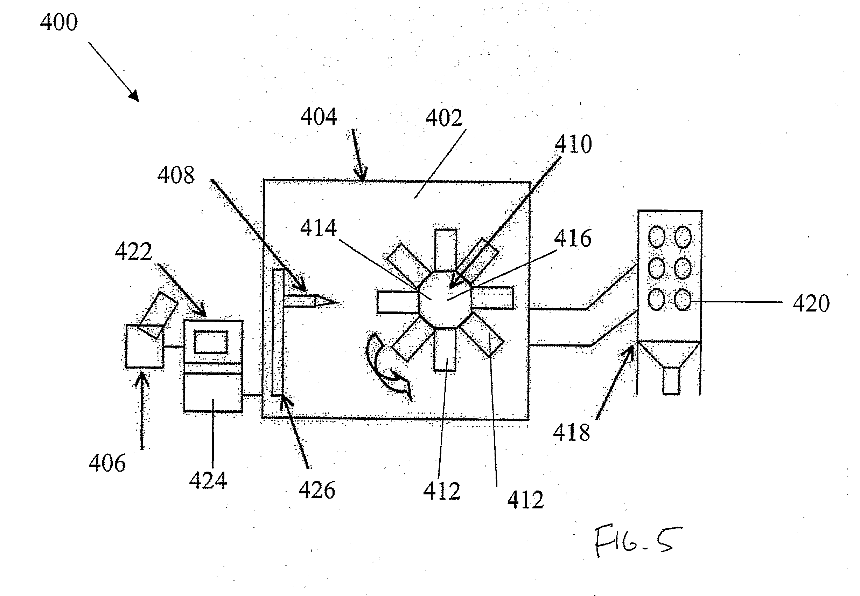

Figure 5 schematically shows a thermal spray system 400 similar to system 300 of Figs. 3 and 4, except system 400 of Figure 5 is adapted for automated processing of larger batches of substrates (not shown) in a protected environment 402 defined by housing 404. Particles, e.g., a fine powder, of a coating composition are supplied from a composition feedstock supply 406 to the thermal spray gun 408. A carrier gas supply (not shown) and heat energy source (not shown) such as fuel, electricity, or the like, are also coupled to gun 408. As shown, gun 408 is a plasma gun, facilitating thermal spraying of materials such as tungsten, which become molten at very high temperatures, e.g., temperatures above about 3400 C. Supply 406 preferably includes an automated powder feeder that is outside environment 402 to facilitate convenient loading of powder feedstock. Gun 408 is generally aimed toward rotatable substrate mounting platform 410 including a plurality of arms 412 extending from centrally positioned rotor 414. Platform 410 rotates about central axis 416. System 400 also includes an exhaust system 418 includes a powder particulate collection system 420.

The movement of both gun 408 and rotatable substrate mounting platform 410 are automated and controlled via computer 422. An operator interfaces with the computer 422 and system 400 via console 424. Rotor 414 desirably has at least a computer-controlled rotation rate and rotation direction. Gun 408 is mounted on robotic manipulation system 426 which can control the distance between gun 408 and arms 412, the height of gun 408 relative to platform 410, the position of gun 408 relative to central axis 416, and the relative angle at which material is sprayed toward platform 410. The supply 406 of material to gun 408 is also automated and may be held constant or varied during the course of a coating operation as desired.

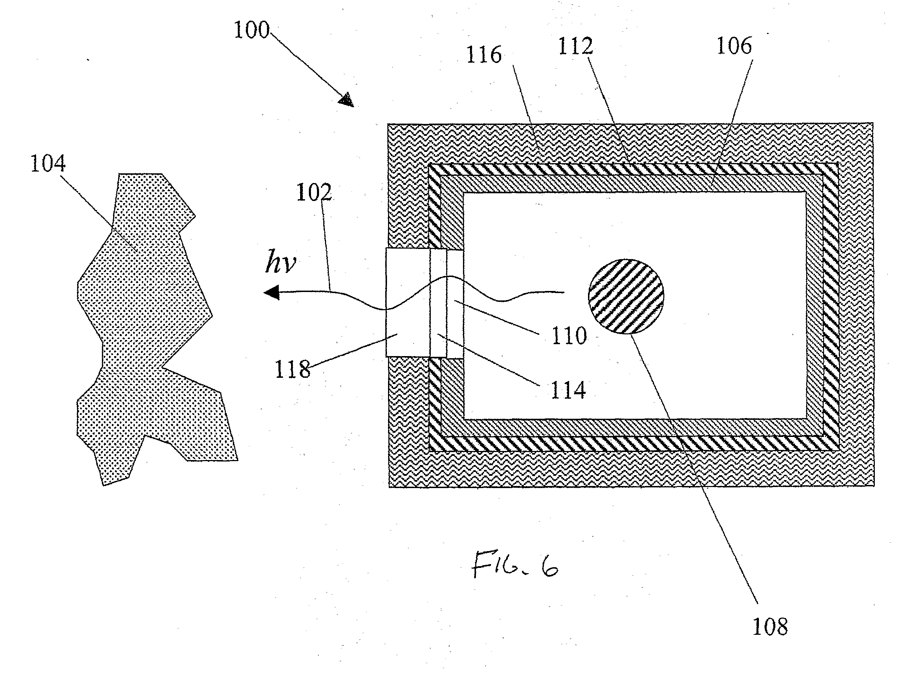

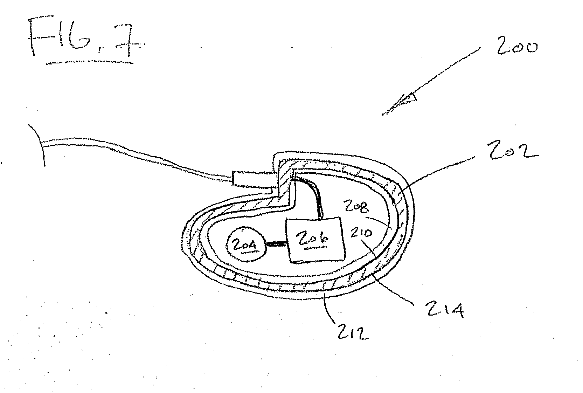

In a typical coating operation, the desired coating material is loaded into automated powder feeder of supply 406. One or more substrates (not shown) to be coated are positioned on one or more of arms 412. Desirably, the substrates are positioned in a balanced manner so that platform 410 rotates smoothly. Thus, pairs of substrates may be positioned in balanced fashion on opposed arms 412 symmetrically about central axis 416. If an odd number of substrates is being processed, a dummy substrate may also be used for balance. As is the case with arms 318 of apparatus 300 of Figs. 6 and 7, arms 412 of system 400 act as a heat sink to help draw thermal energy away from substrates being coated. Cooling media (not shown) desirably also is circulated through arms 412 to help cool the substrates.

The powder is supplied to gun 408 and is sprayed from gun 408 toward rotating platform 410. During spraying, platform 410 rotates at a suitable rotational speed, such as a speed in the range of 100 to 500 rpm. Typically, gun 408 may be indexed radially back and forth relative to platform 410 to help ensure full coverage of surfaces to be coated. The speed at which gun 408 is indexed may be adjusted based upon the position of gun 408 relative to central axis 416 so that coating coverage is uniform notwithstanding the changing relative speed between arms 412 and gun 408 as the radial position of gun 408 with respect to central axis 416 changes. The heat source, in this case a plasma, provides enough heat energy to melt the sprayed particles. Typically, the heat source provides a suitable temperature in the range of 7000 C to about 20000 C. The carrier gas helps to transport the sprayed particles to the substrates. The carrier gas may be any gas such as nitrogen, carbon dioxide, argon, air, combinations of these, and the like. A preferred carrier gas comprises argon and optionally at least one other gas such as hydrogen, helium, nitrogen, carbon dioxide, or the like. A gas such as argon is favored because argon heats quickly in the flame of the gun 408.

A typical supply pressure for the carrier gas is in the range of 30 psi. The preferred primary (argon) and secondary gas (hydrogen) pressures are 75 psi and 50 psi respectively. The molten particles impact on the substrates, where they coalesce and form a coating. Areas of the substrates may be masked if those areas are desired to be uncoated after treatment. A suitable process time may be in the range of a few seconds to 600 seconds or more. A satisfactory coating thickness generally would be in the range of 5 micrometers to about 400 micrometers. Excess spray material is exhausted through exhaust system 418, where entrained particles in the exhaust are collected.

Methods and equipment used to carry out thermal spraying suitable in the practice of the present invention also have been described in U.S. Pat. Nos. 5,762,711; 5,877,093; 6,110,537; 6,287,985; and 6,319,740. Each of these patent documents is incorporated herein by reference.

Figure 6 schematically shows a representative embodiment of an implantable medical device 100 intended to emit focused radiation 102 onto a substrate to be targeted for treatment. Shielding an implantable medical device in this way can also be used to prevent all or some portion of undesirable radiation or energy from leaving the implantable medical device. For purposes of illustration, the substrate is a tumor/cancer growth 104. Device 100 generally includes an envelope 106 housing a radiation source 108. The source 108 may emit radiation naturally and/or the radiation output may be electronically generated. Combinations of energy sources may also be used. Focused radiation 102 is emitted toward growth 104 through radiation transmissive port 110. To help contain off-focus radiation, device 100 is shielded by coating 112 in accordance with principles of the present invention. Coating 112 includes port 114 to allow passage of the focused radiation 102.

Biocompatible layer 116 encapsulates coating 112, and layer 116 also includes a port 118 to allow passage of the focused radiation 102. Advantageously, the shield coating 112 tends to be naturally rough when applied using thermal spray techniques. This provides an excellent surface to which biocompatible layer 116 may adhere. At least that portion of the surface of biocompatible layer 116 proximal to ports 110, 114, and 118 may be polished as appropriate.

Figure 7 schematically shows a cardiac pacemaker 200 similar to the pacemaker 10 described above. Pacemaker 200 includes housing 202, which contains a battery 204 and associated electronic circuitry 206. To help shield electronic circuitry 206 from incoming radiation, radio-opaque coating 208 providing radiation shielding capabilities is preferably provided on inside surface 210 of housing 202 of pacemaker 200. Radio-opaque coating 208 preferably encloses circuitry 206 by coating substantially the entire inside surface 210 of housing 202 of pacemaker 200. Radio-opaque coating 208 can be provided on any internal portion or component of pacemaker 200 such as on an enclosure or the like for electronic circuitry 206 or any portion of pacemaker desired to be shielded in accordance with the present invention. As shown, pacemaker 200 also includes optional biocompatible coating 212 on outside surface 214 of housing 202.

The present invention has now been described with reference to several embodiments thereof. The entire disclosure of any patent or patent application identified herein is hereby incorporated by reference. The foregoing detailed description and examples have been given for clarity of understanding only. No unnecessary limitations are to be understood therefrom. It will be apparent to those skilled in the art that many changes can be made in the embodiments described without departing from the scope of the invention. Thus, the scope of the present invention should not be limited to the structures described herein, but only by the structures described by the language of the claims and the equivalents of those structures.

## Claims

1. A method of integrating a radiation shield with an implantable medical device, the method comprising the steps of providing an implantable medical device and thermally spraying a radio-opaque composition onto a portion of the medical device.

2. The method of claim 1, wherein the implantable medical device comprises active circuitry.

3. The method of claim 2, wherein the radio-opaque composition is thermally sprayed to form a radio-opaque coating that substantially surrounds the active circuitry.

4. The method of claim 3, wherein the radio-opaque coating is external to a housing portion of the implantable medical device.

5. The method of claim 3, wherein the radio-opaque coating is internal to a housing portion of the implantable medical device.

6. The method of claim 1, wherein the implantable medical device comprises one of a pacemaker, defibrillator, nerve stimulator, hearing aid, and drug pump.

7. The method of claim 1, wherein the shielding composition comprises tungsten.

8. The method of claim 1, comprising thermally spraying a biocompatible composition onto a portion of the medical device.

9. The method claim 8, comprising thermally spraying the biocompatible composition onto at least a portion of the radio-opaque composition.

10. The method of claim 8, wherein the biocompatible composition comprises at least one of bioglass, hydroxyapatite, titanium, titanium alloys, alpha-alumina, stabilized zirconia, and apatite ceramics.

11. A method of integrating a radiation shield with a medical device, the method comprising the steps of providing a medical device, thermally spraying a radio-opaque composition onto a first portion of the medical device and thermally spraying a biocompatible composition onto a second portion of the medical device.

12. The method of claim 11, wherein the first portion of the medical device comprises a housing of the medical device.

13. The method of claim 11, wherein the second portion of the medical device comprises a housing of the medical device.

14. The method of claim 11, wherein the radio-opaque composition is thermally sprayed to form a coating external to a housing portion of the implantable medical device.

15. The method of claim 11, wherein the radio-opaque composition is thermally sprayed to form a coating internal to a housing portion of the implantable medical device.
